# EUROPEAN PATENT SPECIFICATION

(11) **EP 0 527 784 B1**
(45) Date of publication and mention of the grant of the patent: **21.08.1996**
(21) Application number: 91908073.9
(22) Date of filing: 11.04.1991
(51) Int. Cl.: C12Q 1/68, G01N 33/36

(54) **DIAGNOSTIC TECHNIQUE**
DIAGNOSTISCHE TECHNIK
TECHNIQUE DIAGNOSTIQUE

(30) Priority: 04.05.1990 GB 9010093
(43) Date of publication of application: 24.02.1993
(73) Proprietor: BRITISH TEXTILE TECHNOLOGY GROUP, Didsbury, Manchester M20 8RX (GB)
(72) Inventor: McCARTHY, Brian, Joseph 12 Parnell Avenue, Manchester M22 4BX (GB); NELSON, Gordon, Manchester M14 6RU (GB); HAMLYN, Paul, Manchester M20 0GF (GB)
(74) Representative: McNeight, David Leslie
(86) International application number: GB9100575
(87) International publication number: WO9117263

(56) References cited:
- DE-A- 3 724 488
- JOURNAL OF EMBRYOLOGY & EXPERIMENTAL MORPHOLOGY, vol. 73, February 1983, The Company of Biologists Ltd., Scarborough, North Yorkshire (GB); L.D. SIRACUSA et al., pp. 163-178
- THE EMBO JOURNAL, vol. 9, no. 5, May 1990, Oxford University Press, Oxford (GB); B.C. POWELL et al., pp. 1585-1493
- GENE ANALYSIS TECHNIQUES, vol. 4, no. 5, 1987, Elsevier Science Publishing Co. Inc., New York, NY (US); J. BRESSER et al., pp. 89-104
- JOURNAL OF CELL BIOLOGY, vol. 110, February 1990, The Rockefeller University Press, New York, NY (US); M.J. FIETZ et al., pp. 427-436

## Description

This invention relates to methods of diagnosis which use molecular biology techniques.

Poly - or oligonucleotide probes (hereinafter "probes") comprising a nucleotide sequence which is hybridisable with RNA or single stranded DNA (hereinafter "genetic material") within a cell or specimen are routinely used in diagnosis.

The degree of preparation of the genetic material varies, but typically involves its isolation from the cell containing it, and frequently, a variable amount of cleavage and electrophoretic separation.

It is not absolutely necessary to isolate genetic material from cells prior to screening with probes. Indeed, prokaryotes may simply be washed, or otherwise placed, onto a support and then subjected to treatments which lyse them in situ to expose genetic material. These cells have a relatively high genetic material content, and notwithstanding the relative simplicity of technique accruing from dispensing with isolation of the material proper, it is preferred that at least the protein component of the cells is removed before application of the probe.

Substantial and/or variable losses in genetic material occur in textile processing and during its isolation, and significant such losses in material occur even when just the protein component is removed from cells.

The problem of substantial and/or variable loss of genetic material from a textile, for example, which comprises more than a single type of fibre means that specific ratios of the fibres in blends thereof cannot be determined by hitherto used molecular-biology techniques.

Powell, B.C. and Rogers, G.E., 1990, EMBO J., 9: 1485-1493 discloses the detection of a sheep wool transgene in tissue sections of mouse dorsal skin biopsies. Fietz, M.J. *et al*.,1990, J. Cell Biol., 110: 427-436 discloses localisation of mRNA in sheep skin sections. Bresser, J. and Evinger-Hodges, M.J., 1987, Gene Anal. Techn., 4: 89-104 discloses various methods of *in situ* hybridisation of mRNA in cells and tissue sections, the cells having had their structural integrity destroyed. DE-A-3724488 discloses the detection of DNA from non-metabolically active biological material comprising degrading the biological material (excluding any DNA) and detecting any DNA present. All of these methods, although involving *'in situ*' hybridisation, involve the step of destroying cellular structural integrity in order to make accessible any nucleic acids. As such they all differ significantly from the present invention which allows the detection of genetic material whilst maintaining, or substantially maintaining, cellular structural integrity.

The present invention provides a method of diagnosis using molecular biology techniques which, at least to some extent, overcomes the disadvantages characterised by prior such methods.

According to the present invention there is provided a method to determine the source of a multicellular biological structure, comprising hybridising a species-specific probe to single stranded genetic material in situ within said structure whilst maintaining, or substantially maintaining, the integrity thereof.

The genetic material may be DNA.

The method may comprise the steps of: accessing double stranded genetic material within the structure, rendering said genetic material single stranded, rendering unreactive groups within the structure that react with a polynucleotide probe other than likewise reactive groups comprised by said genetic material, hybridising said probe with the single stranded genetic material and screening the biological structure for hybridisation.

The structure may be, for example, hair or fur.

Such hair or fur may be from quadrupeds such as the cashmere goat, yak, and llama, for example.

The genetic material in the structure may be DNA which may be accessed by a limited digestion of the structure with proteinases.

The genetic material may be accessed by immersing the structure in an aqueous solution.

The solution may comprise a reducing agent which may be 2-mercaptoethanol or dithiothreitol.

The solution may comprise ethylenediaminetetraacetic acid (EDTA).

The solution may comprise a detergent which may be sodium or lithium dodecyl sulphate.

Alternatively, or additionally, the genetic material may be accessed by sonicating or otherwise mechanically agitating the structure.

The genetic material may be rendered single stranded by known means including denaturing same by incubating the structure in alkali or by subjecting it to molecular excitation, as for example, by microwave irradiation.

Groups (hereinafer "said groups") within the structure which react with the polynucleotide probe, other than likewise reactive groups comprised by said genetic material, may be rendered unreactive by conventional means including incubating the structure with sonicated salmon testes DNA and/or urea.

The positive charge density on said groups may be reduced by increasing the pH within the structure.

The polynucleotide probe may be attached to an enzyme which is chosen having regard to the use of known enzyme-linked reactions whereby ultimately a substrate may be converted into a quantifiable and/or quantitatable product.

The probe may be conjugated to a protein. Following hybridisation, the structure may be incubated with antibodies which are directed against the protein, and which are conjugated to an enzyme which converts a substrate into a quantifiable and/or quantitatable product.

The probe may be conjugated to a fluorescent dye.

Specific probes may be conjugated with specific fluorescent dyes and thus a plurality of probes conjugated to dyes having a plurality of fluorescence maxima may be provided, whereby the fluorescence maxima are used to identify the probes when they are located within structures.

The fluorescent dyes may, for example, be rhodamine, fluorescein, dansyl chloride, fluorescamine, fluoreceinamine, sulphurhodamine, tetramethylrhodamineisothiocyanate, and fluorescent derivatives thereof.

Hybridisation of a radioactive probe to the single stranded genetic material may be determined autoradiographically.

Hybridisation may be determined by an enhanced chemiluminescent technique, in which the probe is cross linked to horseradish peroxidase which in the presence of hydrogen peroxide converts luminol into 3-aminophthalate. In the presence of enhancers the aminophthalate exhibits an emission maximum at 428 nm which can be detected by film sensitive to blue light.

The probe may be RNA or single stranded DNA obtained directly, or otherwise derived, from said quadrupeds.

The probe may be synthetic.

The synthetic probe may hybridise to and recognise the expression of a particular gene or genes in the cells comprised by the structure.

The particular gene or genes may encode keratins. Gene polymorphism may be considered in the preparation of the probe, so that a multiplicity of different probes may be generated so as to be capable of hybridising with genetic material encoding ostensibly a single family of proteins.

The probe may likewise be synthesised having regard to the genome of the animal from which the structure is derived.

The invention will be further apparent from the following description, which illustrates the technique according to the invention, by way of example only, and with reference to the detection of goat-derived textile fibres in a fabric comprising a blend of fibres, some derived from goat and others from yak.

The technique for detecting species specific genetic material in situ within a multi-cellular structure, comprises a plurality of steps.

The DNA within the putative goat fibre is accessed by a limited digestion thereof with proteinases such as the fungal type XI proteinase from Tritirachium album, and the fibre is typically incubated in a solution comprising between 0.1 and 0.5% (w/v) thereof depending on the desired result.

It will be appreciated that other proteinases can be used, depending on the identity of the biological structure and degree of access required.

Under appropriate conditions the solution contains a reducing agent such as 2-mercaptoethanol or dithiothreitol.

The solution can also contain EDTA and/or a detergent such as the sodium or lithium salt of dodecyl sulphate. The detergent is present at 0.1 to 1% (w/v) and the fibre is incubated therein typically overnight.

Alternatively, or additionally, the DNA might be accessed by sonicating or otherwise mechanically agitating the fibre.

The DNA within the fibre is rendered single stranded by denaturation in alkali, although other techniques including subjecting it to molecular excitation, as for example, by microwave irradiation may, under certain circumstances, be more appropriate.

Positively charged groups on, for example, amino acids, such as the guanidino group on arginine or epsilon amino group on lysine, may react with the negatively charged poly or oligo nucleotide probe, resulting in high backgrounds.

These groups are relatively deactivated by incubating the fibre with sonicated salmon testes DNA and/or urea.

Alternatively and/or additionally the positive charge density on the groups is reduced by increasing the pH within the fibre.

A probe is prepared comprising a polynucleotide sequence which is complementary to a sequence of single stranded DNA within the fibre.

The probe comprises single stranded DNA obtained directly, or otherwise derived, from the species of goat from which the fibre is derived.

It will be appreciated, however, that a synthetic probe can equally well be used and may have the advantage that its synthesis may take cognizance of the expression of a particular gene or genes in the cells comprised by the fibre.

These genes may for example encode keratins, and the probe may likewise be synthesised having regard to the genome of the animal from which the fibre is derived.

However the probe is obtained it is rendered radioactive (for example in a nick translation system) and hybridised with the DNA within the fibre.

Suitable washing regimes are performed and the hybridisation detected autoradiographically.

Where the fibre is derived from other than the species of goat aforementioned, the DNA therein will not hybridise with the probe specific for the goat species and no, or little, radiation will be detected by the X-ray film used in the autoradiographic step.

In view of the foregoing, however, it will be now appreciated that the probe need not neccessarily be radioactive, but might be attached to an enzyme which is chosen having regard to the use of known enzyme linked reactions whereby ultimately a substrate is converted into a quantifiable and/or quantitatable product.

The probe could be conjugated to a protein. Following hybridisation, the fibre may be incubated with antibodies which are directed against the protein, and which are conjugated to an enzyme which converts a substrate into a quantifiable and/or quantitatable product.

The probe may be conjugated to a fluorescent dye.

Specific probes may be conjugated with specific fluorescent dyes and thus a plurality of probes conjugated to dyes having a plurality of fluorescence maxima may be provided, whereby the fluorescence maxima are used to identify the probes when they are located within fibres.

The fluorescent dyes may, for example, be rhodamine, fluorescein, dansyl chloride, fluorescamine, fluoreceinamine, sulphurhodamine, tetramethylrhodamine isothiocyanate, and fluorescent derivatives thereof.

Thus a probe specific for yak DNA may be labelled with rhodamine which fluoresces red under UV light, and a second probe specific for goat DNA may be labelled with fluorescein which fluoresces green when subjected to UV light so that yak and goat derived fibres may, after the hybridisation step, be recognised simply from the colour of fluorescence as visualised under a UV microscope.

Alternatively the hybridisation may be determined by an enhanced chemiluminescent technique, in which the probe is cross linked to horseradish peroxidase which in the presence of hydrogen peroxide converts luminol into 3-aminophthalate. In the presence of enhancers the aminophthalate exhibits an emission maximum at 428 nm which can be detected by film sensitive to blue light.

For example, the genetic material within the structure under test may be amplified by any known means including the polymerase chain reaction method.

## Claims

1. A method to determine the source of a multi-cellular biological structure, comprising hybridising a species-specific probe to single stranded genetic material in situ within said structure whilst maintaining, or substantially maintaining, the integrity thereof.

2. A method according to claim 1, in which the genetic material comprises DNA.

3. A method according to claim 1, comprising the steps of: accessing double stranded genetic material within the structure, rendering said genetic material single stranded, rendering unreactive groups within the structure that react with a polynucleotide probe other than likewise reactive groups comprised by said genetic material, hybridising said probe with the single stranded genetic material and screening the biological-structure for hybridisation.

4. A method according to claim 1, in which the structure is hair or fur.

5. A method according to claim 4, in which the hair or fur is from a quadruped.

6. A method according to claim 5, used to distinguish between hair of different quadrupeds.

7. A method according to claim 6, wherein the quadrupeds include cashmere goat, yak and llama.

8. A method according to claim 2, in which the DNA is accessed by a limited digestion with proteinases.

9. A method according to any one of claims 1 to 8, in which the genetic material is accessed by immersing the structure in an aqueous solution.

10. A method according to claim 9, in which the said solution comprises a reducing agent.

11. A method according to claim 10, in which the reducing agent comprises 2-mercaptoethanol.

12. A method according to claim 11, in which the reducing agent comprises dithiothreitol.

13. A method according to any one of claims 9 to 12, in which the solution comprises ethyldiaminetetra-acetic acid (EDTA).

14. A method according to any one of claims 9 to 13, in which the solution comprises a detergent.

15. A method according to claim 14, in which the detergent comprises sodium or lithium dodecyl sulphate.

16. A method according to any one of claims 1 to 15, in which the genetic material is accessed by sonicating or otherwise mechanically agitating the structure.

17. A method according to claim 1, in which the genetic material is rendered single-stranded by denaturising the same by incubating the structure in alkali.

18. A method according to claim 1, in which the genetic material is rendered single-stranded by molecular excitation.

19. A method according to claim 18, wherein the molecular excitation is by microwave radiation.

20. A method according to any one of claims 3 to 7, in which said groups within the structure that react with the polynucleotide probe, other than likewise reactive groups comprised by said genetic material, are rendered unreactive by incubating the structure with sonicated salmon testes DNA and/or urea.

21. A method according to claim 20, in which the positive charge density on said groups is reduced by increasing the pH within the structure.

22. A method according to claim 3, in which the polynucleotide probe is attached to an enzyme which is chosen having regard to the use of known enzyme-linked reactions whereby ultimately a substrate is converted into a quantifiable product.

23. A method according to claim 3, in which said probe is conjugated to a protein.

24. A method according to claim 23, in which, following hybridisation, the structure is incubated with antibodies which are directed against the protein and which are conjugated to an enzyme which converts a substrate into a quantifiable product.

25. A method according to claim 3, in which the probe is conjugated to a fluorescent dye.

26. A method according to claim 25, in which a plurality of specific probes conjugated to fluorescent dyes are provided and the fluorescence maxima are used to identify the probes when they are located within the structure.

27. A method according to claim 25 or claim 26, in which the fluorescent dye or dyes is or are selected from rhodamine, fluorescein, dansyl chloride, fluorescamine, fluoreceinamine, sulphurhodamine, tetramethylrhodamine-isothiocyanate, and fluorescent derivatives thereof.

28. A method according to any one of claims 1 to 27, in which hybridisation of a radioactive probe to the single stranded genetic material is determined autoradiographically.

29. A method according to any one of claims 1 to 28, in which hybridisation is determined by an enhanced chemiluminescent technique in which the probe is cross linked to horseradish peroxidase which in the presence of hydrogen peroxide converts luminol into 3-aminophthalate.

30. A method according to any one of claims 1 to 29, in which the probe is RNA or single stranded DNA.

31. A method according to any one of claims 1 to 30, in which the probe is synthetic.

32. A method according to claim 31, in which a multiplicity of different probes is generated so as to be capable of hybridising with genetic material encoding a single family of proteins, taking into account gene polymorphism.

## Patentansprüche

1. Verfahren zur Bestimmung der Quelle einer vielzelligen biologischen Struktur, dadurch gekennzeichnet, daß eine artspezifische Testsubstanz mit einem einsträngigen genetischen Material in situ innerhalb der genannten Struktur hybridisiert wird während ihre Unversehrtheit aufrechterhalten oder im wesentlichen aufrechterhalten wird.

2. Verfahren nach Anspruch 1, bei dem das genetische Material DNA umfaßt.

3. Verfahren nach Anspruch 1, gekennzeichnet durch folgende Schritte:
Zugriff auf das doppelsträngige genetische Material innerhalb der Struktur, Umwandlung des genetischen Materials in Einzelstränge, Inaktivierung von Gruppen innerhalb der Struktur, die mit einer Polynucleotidtestsubstanz reagieren und die von den in ähnlicher Weise reaktiven Gruppen, die Teil des genetischen Materials sind, verschieden sind, Hybridisierung der genannten Testsubstanz mit dem einzelsträngigen genetischen Material und Sichten der biologischen Struktur auf Hybridisierungen.

4. Verfahren nach Anspruch 1, dadurch gekennzeichnet, daß die Struktur Haar oder Pelz ist.

5. Verfahren nach Anspruch 4, dadurch gekennzeichnet, daß das Haar oder der Pelz von einem Vierfüßer ist

6. Verfahren nach Anspruch 5, dadurch gekennzeichnet, daß es zur Unterscheidung zwischen Haaren verschiedener Vierfüßer verwendet wird.

7. Verfahren nach Anspruch 6, dadurch gekennzeichnet, daß die Vierfüßer Kaschmirziege, Yak und Lama umfassen.

8. Verfahren nach Anspruch 2, dadurch gekennzeichnet, daß die DNA durch eine begrenzte Verdauung mit Proteinasen zugänglich gemacht wird.

9. Verfahren nach einem der Ansprüche 1 bis 8, dadurch gekennzeichnet, daß das genetische Material durch Eintauchen der Struktur in eine wässrige Lösung zugänglich gemacht wird.

10. Verfahren nach Anspruch 9, dadurch gekennzeichnet, daß die genannte Lösung ein Reduktionsmittel enthält.

11. Verfahren nach Anspruch 10, dadurch gekennzeichnet, daß das Reduktionsmittel 2-Mercaptoethanol enthält.

12. Verfahren nach Anspruch 11, dadurch gekennzeichnet, daß das Reduktionsmittel Dithiothreitol enthält.

13. Verfahren nach einem der Ansprüche 9 bis 12, dadurch gekennzeichnet, daß die Lösung Ethyldiamin-tetra-Essigsäure (EDTA) enthält.

14. Verfahren nach einem der Ansprüche 9 bis 13, dadurch gekennzeichnet, daß die Lösung ein Detergens enthält.

15. Verfahren nach Anspruch 14, dadurch gekennzeichnet, daß das Detergens Natrium- oder Lithium-dodecylsulfat enthält.

16. Verfahren nach einem der Ansprüche 1 bis 15, dadurch gekennzeichnet, daß das genetische Material durch Ultrabeschallung oder andererseits mechanisches Rühren der Struktur zugänglich gemacht wird.

17. Verfahren nach Anspruch 1, dadurch gekennzeichnet, daß das genetische Material mittels Denaturierung desselben durch Inkubation der Struktur in Alkali einzelsträngig gemacht wird.

18. Verfahren nach Anspruch 1, dadurch gekennzeichnet, daß das genetische Material durch molekulare Anregung einzelsträngig gemacht wird.

19. Verfahren nach Anspruch 18, dadurch gekennzeichnet, daß die molekulare Anregung durch Mikrowellenstrahlung erfolgt.

20. Verfahren nach einem der Ansprüche 3 bis 7, dadurch gekennzeichnet, daß die genannten Gruppen innerhalb der Struktur, die mit der Polynucleotidtestsubstanz reagieren und die von in ähnlicher Weise reaktiven Gruppen, die in dem genannten genetischen Material enthalten sind, verschieden sind, nichtreaktiv gemacht werden durch Inkubation der Struktur mit ultrabeschallter DNA aus Lachstestes und/oder Harnstoff.

21. Verfahren nach Anspruch 20, dadurch gekennzeichnet, daß die positive Ladungsdichte auf den genannten Gruppen durch Erhöhung des pH-Wertes innerhalb der Struktur verringert wird.

22. Verfahren nach Anspruch 3, dadurch gekennzeichnet, daß die Polynucleotidtestsubstanz an ein Enzym gebunden ist, das bezüglich der Benutzung von bekannten enzymgebundenen Reaktionen ausgewählt wurde, bei denen letzlich ein Substrat in ein mengenmäßig bestimmbares Produkt umgewandelt wird.

23. Verfahren nach Anspruch 3, dadurch gekennzeichnet, daß die genannte Testsubstanz an ein Protein gebunden ist.

24. Verfahren nach Anspruch 23, dadurch gekennzeichnet, daß nach der Hybridisierung die Struktur mit Antikörpern inkubiert wird, die gegen das Protein gerichtet sind und die an ein Enzym gebunden sind, das ein Substrat in ein mengenmäßig bestimmbares Produkt umwandelt.

25. Verfahren nach Anspruch 3, dadurch gekennzeichnet, daß die Testsubstanz an einen fluoreszierenden Farbstoff gebunden ist.

26. Verfahren nach Anspruch 25, dadurch gekennzeichnet, daß eine Vielheit von an fluoreszierende Farbstoffe gebundenen, spezifischen Testsubstanzen zur Verfügung gestellt werden und daß die Fluoreszenzmaxima verwendet werden, um die Testsubstanzen zu erkennen, wenn sie innerhalb der Struktur ausfindig gemacht werden.

27. Verfahren nach Anspruch 25 oder Anspruch 26, dadurch gekennzeichnet, daß der fluoreszente Farbstoff oder die fluoreszenten Farbstoffe aus Rhodamin, Fluoreszein, Dansylchlorid, Fluorescamin, Fluoresceinamin, Sulforhodamin, Tetramethylrhodamin-Isothiocyanat und ihren fluoreszenten Abkömmlingen ausgewählt wird oder werden.

28. Verfahren nach einem der Ansprüche 1 bis 27, dadurch gekennzeichnet, daß die Hybridisierung einer radioaktiven Testsubstanz mit dem einzelsträngigen genetischen Material autoradiographisch bestimmt wird.

29. Verfahren nach einem der Ansprüche 1 bis 28, dadurch gekennzeichnet, daß die Hybridisierung durch ein verstärktes chemilumineszentes Verfahren bestimmt wird, bei welchem die Testsubstanz quervernetzt wird mit Meerrettich-Peroxidase, die in der Gegenwart von Wasserstoffperoxid Luminol in 3-Aminophthalat umwandelt.

30. Verfahren nach einem der Ansprüche 1 bis 29, dadurch gekennzeichnet, daß die Testsubstanz RNA oder einzelsträngige DNA ist.

31. Verfahren nach einem der Ansprüche 1 bis 30, dadurch gekennzeichnet, daß die Testsubstanz eine künstliche ist.

32. Verfahren nach Anspruch 31, dadurch gekennzeichnet, daß eine Mehrzahl von verschiedenen Testsubstanzen erzeugt wird, um zur Hybridisierung mit genetischem Material fähig zu sein, das eine einzige Proteinfamilie codiert unter Berücksichtigung von Genpolymorphismus.

## Revendications

1. Procédé pour déterminer l'origine d'une structure biologique multicellulaire, comprenant l'hybridation d'une sonde spécifique d'une espèce à un matériel génétique monobrin in situ au sein de ladite structure, tout en en conservant ou en en conservant sensiblement l'intégrité.

2. Procédé selon la revendication 1, dans lequel le matériel génétique comprend de l'ADN.

3. Procédé selon la revendication 1, comprenant les étapes consistant à : accéder au matériel génétique double brin au sein de la structure, rendre monobrin ledit matériel génétique, rendre non réactifs au sein de la structure des groupements qui réagissent avec une sonde polynucléotidique, autres que des groupements réactifs similaires englobés par ledit matériel génétique, hybrider ladite sonde audit matériel génétique monobrin et cribler la structure biologique pour hybridation.

4. Procédé selon la revendication 1, dans lequel la structure est des poils ou de la fourrure.

5. Procédé selon la revendication 4, dans lequel les poils ou la fourrure proviennent d'un quadrupède.

6. Procédé selon la revendication 5, utilisé pour faire la distinction entre les poils de quadrupèdes différents.

7. Procédé selon la revendication 6, dans lequel les quadrupèdes comprennent la chèvre de Cachemire, le yak et le lama.

8. Procédé selon la revendication 2, dans lequel on accède à l'ADN par digestion limitée par des protéinases.

9. Procédé selon l'une quelconque des revendications 1 à 8, dans lequel on accède au matériel génétique en immergeant la structure dans une solution aqueuse.

10. Procédé selon la revendication 9, dans lequel ladite solution comprend un agent réducteur.

11. Procédé selon la revendication 10, dans lequel l'agent réducteur comprend le 2-mercaptoéthanol.

12. Procédé selon la revendication 11, dans lequel l'agent réducteur comprend le dithiothréitol.

13. Procédé selon l'une quelconque des revendications 9 à 12, dans lequel la solution comprend de l'acide éthyldiaminotétraacétique (EDTA).

14. Procédé selon l'une quelconque des revendications 9 à 13, dans lequel la solution comprend un détergent.

15. Procédé selon la revendication 14, dans lequel le détergent comprend du dodécylsulfate de sodium ou de lithium.

16. Procédé selon l'une quelconque des revendications 1 à 15, dans lequel on accède au matériel génétique par traitement par ultrasons ou autre agitation mécanique de la structure.

17. Procédé selon la revendication 1, dans lequel le matériel génétique est rendu monobrin par sa dénaturation par incubation de la structure dans des alcalis.

18. Procédé selon la revendication 1, dans lequel le matériel génétique est rendu monobrin par excitation moléculaire.

19. Procédé selon la revendication 18, dans lequel l'excitation moléculaire s'effectue par rayonnement micro-ondes.

20. Procédé selon l'une quelconque des revendications 3 à 7, dans lequel lesdits groupements au sein de la structure, réagissant avec la sonde polynucléotidique, autres que des groupements réactifs similaires englobés par ledit matériel génétique, sont rendus non réactifs par incubation de la structure avec de l'ADN de testicules de saumon traités par ultrasons et/ou de l'urée.

21. Procédé selon la revendication 20, dans lequel la densité de la charge positive sur lesdits groupes est réduite par élévation du pH au sein de la structure.

22. Procédé selon la revendication 3, dans lequel la sonde polynucléotidique est fixée à une enzyme choisie en fonction de l'utilisation de réactions enzymatiques connues où un substrat est finalement converti en produit quantifiable.

23. Procédé selon la revendication 3, dans lequel ladite sonde est conjuguée à une protéine.

24. Procédé selon la revendication 23, dans lequel, après l'hybridation, la structure est incubée avec des anticorps dirigés contre la protéine et qui sont conjugués à une enzyme qui convertit un substrat en produit quantifiable.

25. Procédé selon la revendication 3, dans lequel la sonde est conjuguée à un colorant fluorescent.

26. Procédé selon la revendication 25, dans lequel est fournie une pluralité de sondes spécifiques conjuguées à des colorants fluorescents et on utilise les maxima de fluorescence sont utilisés pour identifier les sondes lorsqu'elles sont situées au sein de la structure.

27. Procédé selon la revendication 25 ou 26, dans lequel le ou les colorants fluorescents sont sélectionnés parmi la rhodamine, la fluorescéine, le chlorure de dansyle, la fluorescamine, la fluorescéinamine, la sulfurhodamine, l'isothiocyanate de tétraméthylrhodamine et leurs dérivés fluorescents.

28. Procédé selon l'une quelconque des revendications 1 à 27, dans lequel l'hybridation d'une sonde radioative au matériel génétique monobrin est déterminée par autoradiographie.

29. Procédé selon l'une quelconque des revendications 1 à 28, dans lequel l'hybridation est déterminée par une technique de chimioluminescence amplifiée où la sonde est réticulée à de la peroxydase de raifort qui, en présence de peroxyde d'hydrogène, convertit le luminol en 3-aminophtalate.

30. Procédé selon l'une quelconque des revendications 1 à 29, dans lequel la sonde est de l'ARN ou de l'ADN monobrin.

31. Procédé selon l'une quelconque des revendications 1 à 30, dans lequel la sonde est synthétique.

32. Procédé selon la revendication 31, dans lequel on crée une multiplicité de sondes différentes pour qu'elles soient capables de s'hybrider à du matériel génétique codant une seul famille de protéines, compte tenu du polymorphisme génique.
